# EUROPÄISCHE PATENTSCHRIFT

(11) **EP 2 603 482 B1**
(45) Veröffentlichungstag und Bekanntmachung des Hinweises auf die Patenterteilung: **01.10.2014**
(21) Anmeldenummer: 11748262.0
(22) Anmeldetag: 28.07.2011
(51) Int. Cl.: C07C 29/10, C07C 29/80, C07C 29/90, C07C 41/42, C07C 41/44, C07C 29/74, C07C 41/34

(54) **VERFAHREN ZUR GEWINNUNG VON DI-TRIMETHYLOLPROPAN UND MIT TRIMETHYLOLPROPAN ANGEREICHERTEN PRODUKTSTRÖMEN AUS DEN NEBENSTRÖMEN DER TRIMETHYLOLPROPANHERSTELLUNG**
METHOD FOR OBTAINING DITRIMETHYLOLPROPANE AND TRIMETHYLOLPROPANE-ENRICHED PRODUCT STREAMS FROM THE SIDE-STREAMS IN TRIMETHYLOLPROPANE PRODUCTION
PROCÉDÉ D'OBTENTION DE DI(TRIMÉTHYLOLPROPANE) ET DE FLUX DE PRODUITS ENRICHIS EN TRIMÉTHYLOLPROPANE À PARTIR DES FLUX DE SOUS-PRODUITS DE LA PRODUCTION DE TRIMÉTHYLOLPROPANE

(30) Priorität: 11.08.2010 DE 102010033844
(43) Veröffentlichungstag der Anmeldung: 19.06.2013
(73) Patentinhaber: OXEA GmbH, 46147 Oberhausen (DE)
(72) Erfinder: KREICKMANN, Thorsten, 46149 Oberhausen (DE); FREY, Guido, D., 64560 Riedstadt (DE); NOWOTNY, Norman, 45276 Essen (DE); STRUTZ, Heinz, 47445 Moers (DE)
(86) Internationale Anmeldenummer: PCT/EP2011/003788
(87) Internationale Veröffentlichungsnummer: WO 2012/019714

(56) Entgegenhaltungen:
- WO-A1-2004/013074
- DE-A1- 19 840 276
- DE-A1-102008 038 021
- US-A- 3 962 347

## Beschreibung

Die vorliegende Erfindung betrifft ein Verfahren zur Gewinnung von Di-Trimethylolpropan und mit Trimethylolpropan angereicherten Produktströmen aus den Nebenströmen der Trimethylolpropanherstellung.

Trimethylolpropan ist ein dreiwertiger Alkohol, der für die Herstellung von Lacken, Polyurethanen und Polyestern, wie beispielsweise von Alkydharzen, von Bedeutung ist. Industriell wird Trimethylolpropan durch Kondensationsreaktion von n-Butyraldehyd mit Formaldehyd nach verschiedenen Varianten hergestellt.

Bei dem sogenannten Hydrierverfahren addiert man zumindest zwei Mole Formaldehyd mit einem Mol n-Butyraldehyd in Gegenwart einer katalytischen Menge eines tertiären Amins über die Zwischenstufe Monomethylolbutyraldehyd zunächst zum Dimethylolbutyraldehyd, der anschließend in einem Hydrierschritt zum Trimethylolpropan umgesetzt wird. Nach dem in WO98/28253 A1 beschriebenen Verfahren wird Formaldehyd mit bis zu einem achtfachen molaren Überschuss eingesetzt. Das erhaltene Reaktionsgemisch aus dem Aldoladditionsschritt wird entweder destillativ oder durch Phasentrennung aufgearbeitet. Bei der destillativen Aufarbeitung werden nicht umgesetzte oder teilumgesetzte Ausgangsverbindungen als flüchtige Komponenten abgezogen und in die Reaktionsstufe zurückgeführt, während das Sumpfprodukt weiter umgesetzt wird. Wird anstelle der destillativen Aufarbeitung das Reaktionsgemisch in einem Phasentrenner in die wässrige und organische Phase geschieden, wird die organische Phase in die Aldoladdition zurückgefahren und die wässrige Phase weiter verarbeitet. Es folgt eine katalytische und/oder thermische Behandlung, um Monomethylolbutyraldehyd in Dimethylolbutyraldehyd zu überführen. Hierbei gebildete Nebenprodukte werden destillativ abgetrennt und das Sumpfprodukt dieser Destillation wird anschließend katalytisch hydriert, um Trimethylolpropan zu gewinnen. Das erhaltene rohe Trimethylolpropan wird anschließend einer Reindestillation unterzogen. Nach Leicht- und Mittelsiederabtrennung erhält man gereinigtes Trimethylolpropan als Zwischenfraktion, während höhersiedende Kondensationsprodukte, in denen Trimethylolpropanäquivalente gebunden sind, als Nachlauf- oder Sumpffraktion anfallen.

Neben dem Hydrierverfahren wird Trimethylolpropan technisch auch über die sogenannte Cannizzaro-Reaktion hergestellt. In einer ersten Reaktionsstufe lässt man dabei n-Butyraldehyd und Formaldehyd unter Zugabe von stöchiometrischen Mengen einer Base zum Dimethylolbutyraldehyd reagieren, das anschließend mit überschüssigem Formaldehyd zum Trimethylolpropan reduziert wird, während gleichzeitig ein Aquivalent Formiat gebildet wird. Üblicherweise wird als Base eine wässrige Lösung einer Alkalimetall- oder Erdalkalimetallverbindung verwendet, beispielsweise Natrium-, Kalium- oder Calciumhydroxid. Da ein Äquivalent Alkalimetall- oder Erdalkalimetallformiat bei dem Cannizzaro-Verfahren als Koppelprodukt anfällt, hängt die Wirtschaftlichkeit dieser Verfahrensvariante auch von den Vermarktungschancen dieses Koppelprodukts ab. Die Aufarbeitung der erhaltenen, wässrigen Reaktionslösung, die Trimethylolpropan, Alkalimetall- oder Erdalkalimetallformiat und überschüssige Base enthält, erfolgt im Allgemeinen durch Extraktion. Nach Neutralisation der überschüssigen Base wird die wässrige Lösung mit einem organischen Lösungsmittel, beispielsweise mit Ethylacetat extrahiert. Die organische Phase wird von der wässrigen Phase, die die Alkalimetall- oder Erdalkalimetallformiate gelöst enthält, getrennt und nach Entfernung des Extraktionsmittels wird Trimethylolpropan durch Destillation gewonnen. Das erhaltene Trimethylolpropan kann weiteren Reinigungsverfahren unterzogen werden. Nach US 5,603,835 wird zunächst aus erhaltenem Trimethylolpropan eine wässrige Lösung hergestellt, die nochmals mit einem organischen Lösungsmittel, beispielsweise mit Methyl-tertiär-butylether, extrahiert wird. Aus der erhaltenen wässrigen Lösung wird Trimethylolpropan mit einer verbesserten Farbzahl von weniger als 100 APHA Einheiten gewonnen.

Gemäß dem aus US 5,948,943 bekannten Verfahren wird die nach der Cannizzaro-Reaktion erhaltene wässrige, rohe Reaktionslösung bei einer solchen Temperatur mit einem geeigneten organischen Lösungsmittel behandelt, dass nur eine flüssige Phase das Extraktionsgefäß verlässt. Bei der anschließenden Abkühlung außerhalb des Extraktionsgefäßes trennt sich die wässrige von der organischen Phase und aus der wässrigen Phase kann Trimethylolpropan mit einer Farbzahl von weniger als 100 APHA isoliert werden.

Es ist ebenfalls bekannt, die Cannizzzaro-Reaktion mit einer organischen Base, beispielsweise mit einem tertiären Amin, durchzuführen. Nach der aus WO97/17313 A1 bekannten Arbeitsweise wird Formaldehyd mit n-Butyraldehyd in Gegenwart von stöchiometrischen Mengen eines tertiären Amins hergestellt, wobei ein Äquivalent des Ammoniumformiats entsteht. Anschließend wird aus dem Rohgemisch Wasser, überschüssiges tertiäres Amin und überschüssiger Formaldehyd abgetrennt und das verbleibende Gemisch erhitzt. Dabei wird das Ammoniumformiat in das tertiäre Amin und Ameisensäure gespalten, wobei das tertiäre Amin und weitere flüchtige Bestandteile abgetrennt werden und es zur Bildung von Trimethylolpropanformiat kommt. Das abgetrennte tertiäre Amin wird entweder in die Cannizzaro-Stufe zurückgeführt oder als Katalysator für die Umesterung des Trimethylolpropanformiats in einer nachgeschalteten Reaktion mit einem zugesetzten niedrigen aliphatischen Alkohol verwendet. Das dabei freigesetzte Trimethylolpropan wird anschließend aus dem Rohprodukt isoliert.

Unabhängig davon, ob die Herstellung von Trimethylolpropan nach dem Hydrierverfahren unter Verwendung katalytischer Mengen eines tertiären Amins, nach dem Cannizzaro-Verfahren mit molaren Mengen eines tertiären Amins und nachfolgender Umesterung des gebildeten Trimethylolpropanformiats oder nach dem Cannizzaro-Verfahren mit molaren Mengen von Alkalimetall- oder Erdalkalimetallhydroxiden und ihrer extraktiven Abtrennung erfolgt, wird das erhaltene rohe Trimethylolpropan einer ein- oder mehrfachen destillativen Reinigung unterzogen, die aufgrund des hohen Siedepunkts im Unterdruck erfolgt. Nach DE 100 58 303 A1 wird die destillative Aufarbeitung des Trimethylolpropans mit einer Ionenaustauscherbehandlung kombiniert, wobei entweder der Aldolisierungsaustrag oder der Hydrieraustrag vor der destillativen Aufarbeitung mit einem stark basischen Ionenaustauscher in Kontakt gebracht wird.

Bei der destillativen Reinigung fallen schwersiedende Fraktionen mit einem im Vergleich zum Trimethylolpropan höheren Siedepunkt oder Rückstände an, in denen Derivate des Trimethylolpropans vorliegen und aus diesem durch Reaktion mit beispielsweise Methanol, Formaldehyd oder auch mit einem weiteren Molekül Trimethylolpropan in den vorgeschalteten Reaktionen entstanden sind. Unter diesen Derivaten sind besonders formaldehydhaltige Acetale vertreten, die durch das Strukturelement -O-CH₂-O- charakterisiert werden und auch als Formale aufgefasst werden können. Unter den Formalen können folgende lineare und cyclische Formale des Trimethylolpropans strukturell beschrieben werden:

Monocyclisches Formal des Trimethylolpropans:

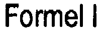

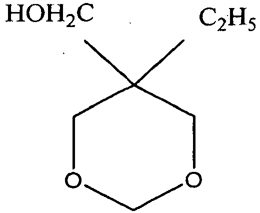

Lineares bis-Trimethylolpropan-Formal:

Formel II [C₂H₅C(CH₂OH)₂CH₂O]₂CH₂

Methyl-(monolineares) Formal des Trimethylolpropans:

Formel III C₂H₅C(CH₂OH)₂CH₂OCH₂OCH₃

Methyl-(bis-lineares) Formal des Trimethylolpropans:

Formel IV C₂H₅C(CH₂OH)₂CH₂OCH₂OCH₂OCH₃

Hierbei siedet das monocycliche Formal des Trimethylolpropans (I) bei einer niedrigeren Temperatur als Trimethylolpropan selbst. Die vom Methanol abgeleiten Formale (III) und (IV) haben einen mit Trimethylolpropan vergleichbaren Siedepunkt während das lineare bis-Trimethylolpropan-Formal (Formel II) als schwersiedende Komponente vorliegt. Darüber hinaus sind noch weitere lineare und cyclische sauerstoffhaltige Verbindungen, wie das cyclische Formal des Di-Trimethylolpropans 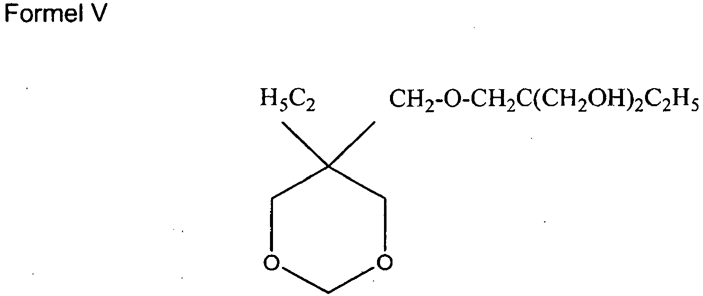 in den Destillationsrückständen zugegen.

Ebenfalls sind in den schwersiedenden Fraktionen und Rückständen der destillativen Aufarbeitung des rohen Trimethylolpropans noch substantielle Mengen an Di-Trimethylolpropan [C₂H₅C(CH₂OH)₂-CH₂-]₂-O und Trimethylolpropan selbst enthalten. Darüber hinaus sind leichtsiedende Komponenten, wie Methanol oder 2-Ethyl-1,3-Propandiol in geringen Mengen anwesend.

Da in den schwersiedenden Fraktionen und Rückständen der destillativen Aufarbeitung des Trimethylolpropans erhebliche Mengen an Derivaten vorliegen, in denen Äquivalente des Trimethylolpropans chemisch gebunden sind, werden eine Reihe von Verfahren vorgeschlagen, um insbesondere formaldehydhaltige Acetale zu spalten und Trimethylolpropan freizusetzen, um auf diese Weise die Ausbeute des gesamten Trimethylolpropanherstellprozesses zu verbessern. Nach WO 2004/013074 A1 werden die bei der Trimethylolpropanherstellung erhaltenen schwersiedenden Fraktionen sowie Destillationsrückstände mit Säure behandelt, wobei der Wassergehalt im Reaktionsansatz 20-90 Gew.-% betragen soll. Aus dem säurebehandelten Produkt kann entweder Trimethylolpropan destillativ gewonnen werden oder das behandelte Produkt kann in die Hydrierstufe des Dimethylolbutyraldehyds zum Trimethylolpropan zurückgeführt werden. Die hydrierende Spaltung von linearen oder cyclischen Acetalen in wässrigen Lösungen in Gegenwart eines heterogenen Hydrierkatalysators zu dem gewünschten mehrwertigen Alkohol ist aus DE 198 40 276 A1 bekannt. Das Verfahren erfordert Hydriertemperaturen oberhalb von 160°C, um den schädlichen Einfluss von Formiaten, die besonders bei dem Arbeiten nach dem Cannizzaro-Verfahren noch zugegen sein können, auf die Hydrierleistung des Katalysators zurückzudrängen. Die hydrierende, katalytische Spaltung kann ebenfalls in Gegenwart einer Säure, beispielsweise in Gegenwart einer niederen Carbonsäure oder sauer reagierender Feststoffe durchgeführt werden.

Die schwersiedenden Fraktionen und die Rückstände der destillativen Aufarbeitung der Trimethylolpropanherstellung enthalten neben den zuvor erwähnten formaldehydhaltigen Acetalen auch bedeutende Mengen an Di-Trimethylolpropan, das ebenfalls als wertvolles Ausgangsmaterial zur Herstellung von Alkydharzen, Weichmachern und Schmiermitteln technische Bedeutung besitzt. Aus dem Stand der Technik sind Verfahren bekannt, Di-Trimethylolpropan aus diesen Rückständen zu gewinnen und so erhaltenes Produkt bei Bedarf weiter zu reinigen.

Nach DE 2058518 A1 wird der Di-Trimethylolpropan haltige Destillationsrückstand einer Wasserdampfdestillation mit überhitztem Wasserdampf unter reduziertem Druck unterzogen. Aus dem erhaltenen wässrigen Destillat wird nach Wasserabtrennung Di-Trimethylolpropan erhalten, das im Bedarfsfalle aus einem organischen Lösungsmittel, beispielsweise Aceton, umkristallisiert werden kann.

Da die destillative Reinigung von Di-Trimethylolpropan infolge des hohen Siedepunktes nur sehr schwer möglich ist und aufgrund der hohen anzuwendenden Temperaturen auch die Zersetzung des Di-Trimethylolpropans zu befürchten ist, wird auch die direkte Aufarbeitung des Destillationsrückstands durch Umkristallisation zur Gewinnung von Di-Trimethylolpropan beschrieben. DE 2358297 A1 behandelt die einfache Kristallisation einer wässrigen Lösung des Destillationsrückstands, wobei in der wässrigen Lösung die Salzkonzentration auf ein besonderes Verhältnis eingestellt wird, um das Ausfällen von Di-Trimethylolpropan in ausreichender Reinheit zu ermöglichen. Wenn Trimethylolpropan nach dem Cannizzaro-Verfahren hergestellt wird, kann der Salzgehalt, beispielsweise der Alkalimetallformiatgehalt in dem Destillationsrückstand bereits genügend hoch sein, um das Ausfallen von Di-Trimethylolpropankristallen in befriedigenderweise nach Auflösung in Wasser sicherzustellen. Gegebenenfalls ist der wässrigen Lösung ein weiteres Salz zuzusetzen, beispielsweise ein Alkalimetallsalz.

US 2004/0254405 A1 offenbart ein Verfahren zur Umkristallisation des Destillationsrückstands unter Verwendung organischer Lösungsmittel, beispielsweise Aceton oder Methylethylketon, wobei die Kristallisationstemperatur, die Lösungsmittelmenge und der Gehalt an Di-Trimethylolpropan im Destillationsrückstand in besonderer Weise einzuhalten sind. Die Verwendung eines Gemisches aus einem geeigneten Lösungsmittel und Wasser für die Isolierung von Di-Trimethylolpropan aus den Destillationsrückständen der Trimethylolpropanherstellung wird in DE 10 2008 038 021 A1 beschrieben. Man erhält zunächst eine organische Lösungsmittelphase und einen viskosen Rückstand, trennt die Phasen und extrahiert die organische Lösungsmittelphase mit Wasser. Die Wasserphase wird abgetrennt und enthaltene Lösungsmittelreste werden entfernt. Aus der verbleibenden Wasserphase wird Di-Trimethylolpropan kristallisiert.

Die bekannten Verfahren zur Gewinnung von Di-Trimethylolpropan aus schwersiedenden Fraktionen und Rückständen, die einen höheren Siedepunkt als Trimethylolpropan aufweisen und die bei der destillativen Aufarbeitung im Rahmen der Trimethylolpropanherstellung anfallen, erfordern entweder aufwendige Umkristallisationsschritte oder eine aufwendige Wasserdampfdestillation mit der nachfolgenden Wasserentfernung aus dem Wasserdampfdestillat.

Es besteht daher ein Bedarf, Di-Trimethylolpropan aus solchen schwersiedenden Fraktionen und Rückständen möglichst einfach mit einer solchen Reinheit zu gewinnen, die für die vorgesehen technischen Anwendungen gefordert wird. Dabei sollen ebenfalls in diesen Fraktionen und Rückständen noch physikalisch eingemischtes Trimethylolpropan sowie darin enthaltene Derivate mit chemisch gebundenen Trimethylölpropaneinheiten als trimethylolpropanreiche Fraktion isoliert werden, die wieder in den Trimethylolpropanreinigungsprozess zurückgeführt werden kann, so dass neben der Gewinnung von reinem Di-Trimethylolpropan auch die Ausbeute an Trimethylolpropan über den gesamten Herstellprozess verbessert werden kann. Auf diese Weise sollen die schwersiedenden Fraktionen und Rückstände, die bei der destillativen Aufarbeitung im Rahmen der Trimethylolpropanherstellung anfallen, möglichst wirtschaftlich genutzt werden.

Die vorliegende Erfindung betrifft daher ein Verfahren zur Gewinnung von Di-Trimethylolpropan und mit Trimethylolpropan angereicherten Produktströmen aus den schwersiedenden Fraktionen und Rückständen, die bei der destillativen Reinigung von Trimethylolpropan anfallen. Es ist dadurch gekennzeichnet, dass man:
(a) diese schwersiedenden Fraktionen und Rückstände vereinigt und unter Wasserzusatz eine wässrige Lösung mit einem Gehalt an organischen Komponenten von 40 bis 90 Gew.-%, bezogen auf die Gesamtmasse, herstellt;
(b) die nach Schritt a) hergestellte wässrige Lösung bei einer Temperatur von 160 bis 280°C und bei einem Druck von 1 bis 30 MPa mit Wasserstoff in Gegenwart eines Katalysators und einer aciden Verbindung, ausgewählt aus der Gruppe von protischen anorganischen Säuren, organischen Säuren und sauren Feststoffen, behandelt;
(c) die nach Schritt b) erhaltene wässrige Lösung vom Katalysator und weiteren Feststoffen, falls vorhanden, abtrennt;
(d) die nach Schritt c) erhaltene wässrige Lösung sowohl mit basischen als auch mit sauren Ionenaustauschern behandelt;
(e) aus dem nach Schritt d) erhaltenen wässrigen Eluat Wasser und vorhandene Leichtsieder entfernt; und
(f) aus dem nach Schritt e) erhaltenen Rückstand bei erhöhter Temperatur und vermindertem Druck einen mit Trimethylolpropan angereicherten Produktstrom abdestilliert, wobei Di-Trimethylolpropan als Destillationsrückstand verbleibt.

Ausgangsprodukte für das erfindungsgemäße Verfahren sind Produktströme, die bei der destillativen Reinigung von Trimethylolpropan anfallen und einen höheren Siedepunkt als Trimethylolpropan aufweisen und als schwersiedende Fraktionen bezeichnet werden können. Neben diesen schwersiedenden aber bei der Destillation noch flüchtigen Komponenten kommt auch der verbleibende Destillationsrückstand in dem erfindungsgemäßen Verfahren zum Einsatz. Diese vereinigten Produktströme enthalten als Hauptkomponenten noch physikalisch eingemischtes Trimethylolpropan, im Allgemeinen in einem Bereich von 5 bis 30 Gew.-%, Di-Trimethylolpropan, im Allgemeinen in einem Bereich von 10 bis 30 Gew.-% sowie das lineare bis-Trimethylolpropan-Formal in einem Bereich von 25 bis 60 Gew.-%, bezogen auf die gesamte Einsatzmasse. Weitere identifizierte Produkte sind 2-Ethyl-1,3-Propandiol und das monocyclische Formal des Trimethylolpropans, die aufgrund ihres vergleichsweise geringen Siedepunkts nur noch in geringen Mengen bis üblicherweise bis zu 2 Gew.-% anwesend sind. Cyclische und lineare Formale darunter das Methyl-(monolineare) Formal des Trimethylolpropans (III), das Methyl-(bis-lineare) Formal des Trimethylolpropans (IV) sowie das cyclische Formal des Di-Trimethylolpropans (V) bilden den Rest der organischen Komponenten in dem Gemisch.

Unabhängig davon, ob Trimethylolpropan nach dem Cannizzaro-Verfahren unter Verwendung von Alkalimetall- oder Erdalkalimetallverbindungen hergestellt wird oder nach dem Hydrierverfahren in Gegenwart katalytischer Mengen von Trialkylaminen oder nach dem Cannizzaro-Verfahren unter Verwendung stöchiometrischer Mengen an Trialkylaminen produziert wird, können die schwersiedenden Fraktionen und die Rückstände, die bei der destillativen Reinigung von Trimethylolpropan nach dem jeweiligen Herstellverfahren anfallen, gemäß der erfindungsgemäßen Arbeitsweise aufgearbeitet werden. Darüber hinaus kann das Einsatzgemisch noch Alkalimetall- oder Erdalkalimetallformiate enthalten, deren Gehalte in Abhängigkeit von der Art des für die Herstellung von Trimethylolpropan angewandten Verfahrens schwanken.

Die schwersiedenden Fraktionen, die einen höheren Siedepunkt als Trimethylolpropan aufweisen, und die Rückstände aus der Destillation von Trimethylolpropan werden vereinigt und mit Wasser unter Bildung einer wässrigen Lösung versetzt. Man stellt eine wässrige Lösung mit einem Gehalt an organischen Komponenten von 40 bis 90 Gew.-%, vorzugsweise von 50 bis 90 Gew.-%, insbesondere von 50 bis 80 Gew.-% und ganz besonders von 60 bis 80 Gew.-% her, bezogen auf die Gesamtmasse. Geringere Gehalte an organischen Komponenten sind aufgrund des hohen Wasseranteils nicht zweckmäßig, bei zu hohen Gehalten ist besonders bei Raumtemperatur mit Inhomogenitäten in der wässrigen Lösung oder mit dem Ausfallen von Feststoffen zu rechnen. Zweckmäßigerweise wird die wässrige Lösung bei einer Temperatur von über 50°C hergestellt. Ein Temperaturbereich für die wässrige Lösung von 60°C bis 80°C ist vorzugsweise einzustellen.

Die erhaltene wässrige Lösung wird anschließend bei erhöhter Temperatur und erhöhtem Druck mit Wasserstoff in Gegenwart eines Katalysators und einer aciden Verbindung behandelt. Man arbeitet bei Temperaturen im Bereich von 160 bis 280°C, vorzugsweise 180 bis 230°C und bei Drücken im Bereich von 1 bis 30 MPa, vorzugsweise 6 bis 20 MPa. Bei den anwesenden aciden Verbindungen handelt es sich um protische anorganische Säuren, organische Säuren oder um saure Feststoffe. Als protische anorganische Säuren kommen Phosphorsäure oder Schwefelsäure in Betracht als organische Säuren niedere Carbonsäuren wie Ameisensäure, Essigsäure, Propionsäure oder die isomeren Buttersäuren. Ihre Menge wird so bemessen, dass die wässrige, der Hydrierung zu unterziehende Lösung einen pH-Wert im Bereich von 1 bis 5, vorzugsweise von 1 bis 3 aufweist. Aufgrund der leichten Abtrennbarkeit ist jedoch das Arbeiten mit sauer reagierenden Feststoffen als acide Verbindung bevorzugt. Als solche Feststoffe eignen sich beispielsweise oxidische Verbindungen wie saures Aluminiumoxid, natürliche oder silikatische Stoffe, wie Mordenit, Montmorillonit oder saure Zeolithe, beispielsweise solche vom Y-Typ, die in technischen Mengen zur Verfügung stehen und beispielsweise bei der katalytischen Spaltung von Rohölen industriell eingesetzt werden. Ihr Zusatz richtet sich nach ihrer Aciditat und je 100 Gew.-Teilen wässriger Lösung werden sie im Allgemeinen in einer Menge von 0,5 bis 2, vorzugsweise von 0,5 bis 1,0 Gew.-Teilen verwendet, wobei die eingesetzten Mengen umso geringer sind je acider der Feststoff ist.

Auch kommerziell verfügbare saure Ionenaustauscher, beispielsweise stark saure Ionenaustauscher wie Amberlyst 15, Amberlite IR 120, Amberlyst DPT-1, Dowex Marathon-C, Dowex HCR oder Lewatit S 100 oder schwach saure Ionenaustauscher wie Amberlite ICR 86 oder Lewatit CNP können eingesetzt werden. Ihr Zusatz richtet sich nach ihrer Acidität und sie werden im Allgemeinen in einer Menge von 1 bis 20, vorzugsweise von 5 bis 10 Gew.-Teilen, bezogen auf 100 Gew.-Teile wässriger Lösung, verwendet, wobei die verwendeten Mengen umso geringer sind je acider der Feststoff ist.

Als Katalysatoren für den Hydrierschritt verwendet man übliche Hydrierkatalysatoren, wobei heterogenen Hydrierkatalysatoren der Vorzug gegeben wird, da sie auf einfache Weise vom Reaktionsgemisch abgetrennt werden können, beispielsweise bei der Suspensionshydrierung durch einfache Filtration. Auch bei fest angeordneten Katalysatoren, beispielsweise bei der Riesel- oder Sumpffahrweise, kann das Reaktionsgemisch vom Hydrierkatalysator leicht separiert werden.

Übliche Hydrierkatalysatoren enthalten als wirksame Komponenten ein Edelmetall aus der Reihe Ru, Rh, Pd oder Pt oder ein Übergangsmetall aus der Reihe Cu, Cr, Co, Ni, Fe und darunter insbesondere Raney-Katalysatoren und Chromit-Katalysatoren. Neben ungeträgerten Katalysatoren kommen auch Trägerkatalysatoren zum Einsatz, insbesondere sind für Ru, Rh, Pd oder Pt geeignete Trägermaterialien Aktivkohle, Aluminiumoxid, SiO₂, TiO₂, ZrO₂ sowie Silikate. Die Metallbeladung bei geträgerten Katalysatoren liegt üblicherweise im Bereich von 0,1 bis 10, vorzugsweise von 1 bis 5 Gew.-%. Als besonders geeignet haben sich Ru, Pd und Pt auf Aktivkohle erwiesen.

Die Hydrierstufe wird in Gegenwart der aciden Verbindung, die entweder in der wässrigen Lösung gelöst vorliegt, beispielsweise im Falle von zugesetzten anorganischen oder niederen organischen Carbonsäuren, oder die als in der wässrigen Lösung suspendierter Festsoff zugegen ist, kontinuierlich oder diskontinuierlich durchgeführt, beispielsweise an fest angeordneten Katalysatoren nach der Rieselfahrweise oder Sumpffahrweise sowie unter Rühren nach der Suspensionshydrierung.

Bei der kontinuierlichen Fahrweise hat sich eine Katalysatorbelastung V/Vh, ausgedrückt in Durchsatzvolumen pro Katalysatorvolumen und Zeit, von 0,1 bis 1 h⁻¹, vorzugsweise von 0,2 bis 0,5 h⁻¹, als zweckmäßig erwiesen. Bei der diskontinuierlichen Verfahrensführung verwendet man, bezogen auf 100 Gew.-Teilen wässrige Einsatzlösung ohne Berücksichtigung der aciden Verbindung, von 0,1 bis 10, vorzugsweise von 0,5 bis 5 Gew.-Teile an Katalysator.

Nach beendeter Hydrierung wird das wässrige Reaktionsgemisch von Feststoffen befreit, beispielsweise durch Filtration, wenn in Gegenwart fester acider Verbindungen hydriert wurde.

Das von Feststoffen befreite Hydriergut wird anschließend abwechselnd mit basischen und sauren kommerziell verfügbaren Ionenaustauschern behandelt, wobei die Reihenfolge der Behandlung keine Rolle spielt. Das Hydriergut wird zunächst mit einem sauren Ionenaustauscher behandelt und das erhaltene Eluat anschließend mit einem basischen Ionenaustauscher in Kontakt gebracht, oder umgekehrt. Man arbeitet bei üblichen Temperaturen im Bereich von 1 bis 100°C, vorzugsweise im Bereich von 20 bis 70°C, und bei Normaldruck. Es hat sich gezeigt, dass die Behandlung mit den Ionenaustauschern wesentlich ist, damit in der nachfolgenden destillativen Aufarbeitung des wässrigen Eluats im Destillationsrückstand Di-Trimethylolpropan mit ausreichender Qualität anfällt. Insbesondere lassen sich die Restmengen an Sulfatasche, bestimmt nach DIN 51575, modifiziert unter Zugabe von Schwefelsäure nach dem Abbrennen und vor dem Glühen der Probe, in dem zurückbleibenden Di-Trimethylolpropan erst durch die Behandlung mit den basischen und sauren Ionenaustauschern deutlich vermindern.

Falls die Hydrierung in Gegenwart gelöster anorganischer Säuren oder niederer organischer Carbonsäuren erfolgte, wird die wässrige Lösung nach Abtrennung des Hydrierkatalyators durch Basenzusatz neutral gestellt. Auch in diesem Falle schließt sich die Behandlung mit einem sauren und basischen Ionenaustauscher, oder umgekehrt an, und zwar bei üblichen Temperaturen im Bereich von 1 bis 100°C, vorzugsweise im Bereich von 20 bis 70°C, und bei Normaldruck. Durch die Behandlung mit den Ionenaustauschern werden nicht nur die nach Basenzusatz gebildeten Salze abgetrennt sondern darüber hinaus noch weitere Verunreinigungen, die für einen erhöhten Sulfatascheanfall im Di-Trimethylolpropan verantwortlich sind.

Zu den basischen Ionenaustauschern zählen solche, die primäre, sekundäre, tertiäre oder quartäre Aminogruppen enthalten. Besondere Bedeutung haben Ionenaustauscher auf Polystyrolbasis, die tertiäre Aminogruppen oder quartäre Aminogruppen in der Basenform enthalten, erlangt. Beispiele für schwach- bis starkbasische Ionenaustauscher sind Amberlit IR 45, Dowex 4 oder Dowex Marathon-A. Besondere technische Bedeutung haben makroretikulare Typen, wie Amberlyst A21, Lewatit MP62, Lewatit MP64, Imac A20, Zerolit G, Amberlit IRA93 oder Amberlyst A26 erlangt.

Schwach oder stark saure Ionenaustauscher enthalten beispielsweise die Carboxylat- oder die Sulfonsäuregruppe, die an eine Polymermatrix auf Basis von Styrol-Divinylbenzol-Copolymeren gebunden sind. Die Carboxylatgruppe kann beispielsweise von aromatischen Carbonsäuren oder aliphatischen Carbonsäuren abgeleitet werden und die Sulfonsäuregruppe von aromatischen oder aliphatischen Sulfonsäuren. Ein stark saurer Ionenaustauscher ist beispielsweise Amberlyst 15, Amberlyst DPT-1 oder Dowex Marathon-C. Man bringt die wässrige Lösung in einem geeigneten Reaktor mit dem Ionenaustauscher in Kontakt. Der Ionenaustauscher kann zum Beispiel in einem Rohrreaktor als Festbett angeordnet werden, durch das die wässrige Lösung strömt. Das Festbettvolumen und die Größe der Ionenaustauscherpartikel können in weiten Bereichen variiert und so den gewählten Reaktionsbedingungen und den Verfahrensgegebenheiten, wie der gewünschten Strömungsgeschwindigkeit, angepasst werden. Es hat sich bewährt, Raumgeschwindigkeiten im Bereich von 1 bis 10, insbesondere von 5 bis 8 (V _{wässr}. _{Lösung} /[V_{Ionenaustausche} . h]) einzuhalten. Es handelt sich hierbei um Richtgrößen, die zweckmäßig zu wählen sind.

Nach einer anderen Ausführungsform der erfindungsgemäßen Arbeitsweise suspendiert man den Ionenaustauscher, der in diesem Fall sehr feinteilig sein kann, in der wässrigen Lösung. Es ist zweckmäßig, die Suspension ständig zu bewegen, zum Beispiel durch Rühren oder Einleiten eines Gases, beispielsweise Luft oder Stickstoff, um den innigen Kontakt zwischen der flüssigen Phase und dem Ionenaustauscher zu erzielen. Das Massenverhältnis von flüssiger Phase zu Ionenaustauscher kann weitgehend frei und somit den individuellen Erfordernissen entsprechend eingestellt werden. Es hat sich bewährt, je 100 Gew.-teile wässriger Lösung 1 bis 10, vorzugsweise 3 bis 8 Gew.-teile Ionenaustauscher einzusetzen. Für die Durchführung dieser Verfahrensvariante eignen sich zum Beispiel Rührkessel oder Autoklaven. Bei dieser Arbeitsweise unterliegt der Ionenaustauscher jedoch einer mechanischen Belastung und für die Vermischung von wässriger Phase mit dem Ionenaustauscher sind die Bedingungen so einzustellen, dass ein Abrieb an der Oberfläche der Teilchen oder gar eine mechanische Schädigung der Teilchen vermieden wird.

Die wässrige Lösung kann rezirkuliert werden, um durch mehrfache Behandlung der wässrigen Phase die Abtrennung von Verunreinigungen zu vervollständigen. Ebenso ist es möglich, die Adsorption in mehreren Stufen durchzuführen, sowohl absatzweise als auch kontinuierliche Reaktionsführung ist möglich.

Nach der Behandlung des wässrigen Hydrieraustrags mit den basischen und sauren Ionenaustauschern wird das erhaltene Eluat destillativ aufgearbeitet. Zunächst werden Leichtsieder, insbesondere Wasser und Methanol, das durch Hydrierung des bei der Acetalspaltung freigesetzten Formaldehyds entstanden ist, als Vorlauf abgetrennt. Die Leichtsiederabtrennung erfolgt üblicherweise bei Sumpftemperaturen von 80 bis 140°C und im leichten Unterdruck bis zu 500 hPa. Nach Abtrennung der überwiegenden Menge an Leichtsiedern wird die Sumpftemperatur auf 180 bis 230°C erhöht und gleichzeitig der Druck bis auf 2 hPa erniedrigt. Man gewinnt als Kopfprodukt einen mit Trimethylolpropan angereicherten Produktstrom mit einem Gehalt in der Größenordung im Bereich um 90 bis 96 Gew.-% Trimethylolpropan. Nach überwiegender Abtrennung des Trimethylolpropans wird die Sumpftemperatur bis auf 260°C angezogen, um letzte Anteile an flüchtigen Komponenten auszutreiben. Im Destillationsrückstand verbleibt Di-Trimethylolpropan als leicht gelbliche Flüssigkeit mit einem gaschromatographisch ermittelten Wertproduktgehalt im Bereich von 96 - 98 Gew.-% in einer für technische Anwendungen ausreichenden Qualität und mit einem deutlich. verminderten Sulfataschegehalt. Nach Abkühlen auf Raumtemperatur erhält man ein nahezu weißes Pulver.

Der abgezogene, mit Trimethylolpropan angereicherte Produktstrom wird in die Reinigungsstufe des Gesamtprozesses für die Herstellung von Trimethylolpropan zurückgeführt, zweckmäßigerweise in die Reindestillationsstufe zur Gewinnung von Trimethylolpropan. Bei den Temperatur- und Druckangaben handelt es sich um Richtwerte, die in routinemäßigerweise optimiert werden können. Um die Zersetzung des Di-Trimethylolpropans im Destillationssumpf zu vermeiden, sollte die Sumpftemperatur aber nicht zu weit über die angegebenen Richtwerte erhöht werden.

Die destillative Aufarbeitung des wässrigen Eluats aus der Behandlung mit den Ionenaustauschern kann sowohl absatzweise als auch kontinuierlich erfolgen. Es eignen sich die in der Technik üblichen Destillationskolonnen, die zweckmäßigerweise von 2 bis 30 Böden besitzen.

Das erfindungsgemäße Verfahren gestattet die wirtschaftliche Verwertung von schwersiedenden Fraktionen und Rückständen, die bei der destillativen Reinigung von Trimethylolpropan anfallen. Durch die Rückführung der daraus gewonnenen trimethylolpropanreichen Produktströme in den gesamten Herstellprozess kann die Anlageneffizienz verbessert und die Ausbeute an Trimethylolpropan um etwa 3 bis 7% verbessert werden gegenüber einer Verfahrensführung, bei der die schwersiedenden Fraktionen und Rückstände aus der Trimethylolpropandestillation nicht aufgearbeitet und nicht zurückgeführt werden. Gleichzeitig fällt bei der erfindungsgemäßen Arbeitsweise Di-Trimethylolpropan als Destillationsrückstand in einer für technische Anwendungen ausreichenden Qualität an, ohne dass eine zusätzliche, nur sehr schwierig durchzuführende Destillation des Di-Trimethylolpropans oder aufwendige Umkristallisationsschritte erforderlich sind.

In den nachfolgenden Beispielen wird das erfindungsgemäße Verfahren näher beschrieben. Es ist selbstverständlich nicht auf die wiedergegebene Ausführungsform beschränkt.

### Beispiele

### Beispiel 1

Für die erfindungsgemäße Aufarbeitung der schwersiedenden Fraktionen und Rückstände aus der destillativen Reinigung von Trimethylolpropan kam ein Gemisch zum Einsatz, das folgende gaschromatographisch ermittelte Zusammensetzung (%) aufwies:

| | |
|---|---|
| Vorlauf | 1,7 |
| Monocyclisches Formal (I) | 0,1 |
| Trimethylolpropan | 19,3 |
| Zwischenlauf I | 3,1 |
| Di-Trimethylolpropan | 20,2 |
| Zwischenlauf II | 16,4 |
| Lineares bis-Trimethylolpropan-Formal (II) | 32,7 |
| Hochsieder | 6,5 |

Aus dem organischen Rückstand wurde unter Zugabe von Wasser bei 60°C eine homogene wässrige Lösung mit einem Gehalt von 60 Gew.-% aufgelöstem organischen Rückstand hergestellt. Zu 100 Gew.-Teilen wässrige Lösung gab man 5 Gew.-Teile eines handelsüblichen Ruthenium-auf-Aktivkohle-Katalysators in Pulverform und mit einer Metallbeiadung von 5 Gew.%, sowie 2 Gew.-Teile eines sauren, handelsüblichen Zeoliths vom Y-Typ. Die erhaltene Suspension wurde anschließend unter folgenden Bedingungen mit Wasserstoff in einem 1 Liter Autoklaven behandelt.

**Tabelle 1: Hydrierung einer wässrigen Lösung von Rückständen aus der Trimethylolpropandestillation an Ruthenium-auf-Aktivkohle in Gegenwart von Zeolith vom Y-Typ**

| Reaktionsbedingungen | Versuch 1 | Versuch 2 | Versuch 3 | Versuch 4 (Vergleich) |
|---|---|---|---|---|
| Temperatur (°C) | 200 | 200 | 200 | 130 |
| Druck (MPa) | 4 | 8 | 20 | 20 |
| Reaktionsdauer (h) | 5 | 5 | 5 | 5 |

| Gaschromatographisch ermittelte Zusammensetzung (%; organischer Anteil, wasserfrei): | | | | |
|---|---|---|---|---|
| Vorlauf | 5,2 | 4,7 | 1,6 | 1,1 |
| Monocycl. Formal (I) | 0,2 | 0,1 | 0,6 | 20,2 |
| Trimethylolpropan | 74,4 | 72,7 | 76,4 | 56,6 |
| Zwischenlauf I | 4,2 | 1,4 | 1,5 | 5,3 |
| Di-Trimethylolpropan | 15,5 | 20,8 | 19,6 | 16,3 |
| Zwischenlauf II | 0,1 | 0,1 | 0,1 | 0,2 |
| Lineares bis- Trimethylolpropan-Formal (II) | 0,1 | 0,1 | 0,1 | 0,1 |
| Hochsieder | 0,3 | 0,1 | 0,1 | 0,2 |

### Beispiel 2

Für die erfindungsgemäße Aufarbeitung der schwersiedenden Fraktionen und Rückstände aus der destillativen Reinigung von Trimethylolpropan kam ein Gemisch zum Einsatz, das folgende gaschromatographisch ermittelte Zusammensetzung (%) aufwies:

| | |
|---|---|
| Vorlauf | 0 |
| Monocyclisches Formal (I) | 0 |
| Trimethylolpropan | 7,0 |
| Zwischenlauf I | 19,8 |
| Di-Trimethylolpropan | 20,3 |
| Zwischenlauf II | 0,1 |
| Lineares bis-Trimethylolpropan-Formal (II) | 47,0 |
| Hochsieder | 5,8 |

Aus dem organischen Rückstand wurde unter Zugabe von Wasser bei 60°C eine homogene wässrige Lösung mit einem Gehalt von 60 Gew.-% aufgelöstem organischen Rückstand hergestellt. Zu 100 Gew.-Teilen wässrige Lösung gab man 0,5 Gew.-Teile eines handelsüblichen Ruthenium-auf-Aktivkohle-Katalysators in Pulverform und mit einer Metallbeiadung von 5 Gew.-%, sowie 0,5 Gew.-Teile eines sauren, handelsüblichen Zeoliths vom Y-Typ. Die erhaltene Suspension wurde anschließend unter folgenden Bedingungen mit Wasserstoff in einem 1 Liter Autoklaven behandelt.

**Tabelle 2: Hydrierung einer wässrigen Lösung von Rückständen aus der Trimethylolpropandestillation an Ruthenium-auf-Aktivkohle in Gegenwart von Zeolith vom Y-Typ**

| Reaktionsbedingungen | Versuch 5 | Versuch 6 | Versuch 7 | Versuch 8 | Versuch 9 |
|---|---|---|---|---|---|
| Temperatur (°C) | 190 | 200 | 210 | 220 | 230 |
| Druck (MPa) | 13 | 13 | 13 | 13 | 13 |
| Reaktionsdauer (h) | 3 | 3 | 3 | 3 | 3 |

| Gaschromatographisch ermittelte Zusammensetzung (%; organischer Anteil, wasserfrei): | | | | | |
|---|---|---|---|---|---|
| Vorlauf | 4,3 | 5,2 | 6,3 | 5,9 | 8,9 |
| Monocycl. Formal (I) | 8,9 | 3,9 | 1,9 | 0,2 | 0,2 |
| Trimethylolpropan | 60,9 | 65,1 | 65,7 | 68,1 | 64,7 |
| Zwischenlauf I | 3,7 | 2,3 | 1,9 | 1,6 | 1,6 |
| Di-Trimethylolpropan | 21,7 | 23,1 | 23,8 | 23,9 | 24,2 |
| Zwischenlauf II | 0,1 | 0,1 | 0,1 | 0,1 | 0,1 |
| Lineares bis- Trimethylolpropan-Formal (II) | 0,1 | 0,1 | 0,1 | 0,1 | 0,1 |
| Hochsieder | 0,3 | 0,2 | 0,2 | 0,1 | 0,2 |

Nach Filtration des Katalysators und des sauren Zeoliths wurde die nach Hydrierung erhaltene wässrige Lösung bei einer Temperatur von 20°C mit einer Belastung V/Vh von 5 h⁻¹ zunächst über ein Bett gefüllt mit dem basischen Ionenaustauscher Dowex Marathon-A und anschließend ebenfalls mit einer Belastung V/Vh von 5 h⁻¹ über ein Bett gefüllt mit dem sauren Ionenaustauscher Dowex Marathon-C gepumpt. Das erhaltene Eluat wurde anschließend an einer üblichen 20 cm Vigreux-Kolonne diskontinuierlich aufdestilliert. Der Verlauf der destillativen Aufarbeitung sowie die gaschromatographische Analyse (%) der einzelnen Fraktionen sowie des Destillationsrückstands sind in der nachfolgenden Tabelle 3 zusammengestellt.

Die Ergebnisse einer kontinuierlich durchgeführten Destillation des erhaltenen wässrigen Eluats sind in Tabelle 4 wiedergegeben. Dabei wurde das Sumpfprodukt der ersten Destillation als Einsatzprodukt für die zweite Destillation verwendet. Für die Destillationen benutzte man mit Raschigringen gefüllte Laborfüllkörperkolonnen mit acht theoretischen Böden bei der ersten Destillation und mit vier theoretischen Böden bei der zweiten Destillation.

**Tabelle 3: Diskontinuierliche destillative Aufarbeitung der wässrigen Lösung von Rückständen aus der Trimethylolpropanherstellung nach Hydrierung und Behandlung mit Ionenaustauschern**

| | Einsatz | 1. Fraktion | 2. Fraktion | 3. Fraktion | 4. Fraktion | 5. Fraktion | 6. Fraktion | Rückstand |
|---|---|---|---|---|---|---|---|---|
| Kopf-Temp. °C | | 98-104 | 20-24 | 132-173 | 178-190 | 210-220 | 220-224 | |
| Sumpf-Temp. °C | | 100-180 | 70-160 | 176-190 | 196-240 | 250 | 250 | |
| Druck hPa | | 1013 | 10 | 10 | 10 | 2 | 2 | |
| Gewicht (Gramm)* | 4662,4 | 1980,1 | 16,5 | 957,9 | 967,1 | 296,6 | 61,0 | 353,9 |
| %-Anteil | | 42,5 | 0,4 | 20,5 | 20,7 | 6,4 | 1,3 | 7,6 |
| Wassergehalt (%) | 40 | 95 | | | | | | |

| Gaschromatographische Analyse (%): | | | | | | | | |
|---|---|---|---|---|---|---|---|---|
| Vorlauf | 1,2 | | 0,8 | 0 | 0 | 0 | 0 | 0 |
| Monocycl. Formal (I) | 5,1 | | 57,8 | 6,6 | 0,1 | 0,2 | 0,1 | 0,1 |
| Trimethylolpropan | 77,8 | | 36,2 | 92,3 | 98,5 | 87,0 | 2,4 | 0,1 |
| Zwischenlauf I | 1,0 | | 5,2 | 0,9 | 0,5 | 3,5 | 6,2 | 0,4 |
| Di-Trimethylolpropan | 14.9 | | 0 | 0,2 | 0,9 | 9,2 | 90,4 | 97,4 |
| Zwischenlauf II | 0 | | 0 | 0 | 0 | 0 | 0 | 0,1 |
| Lineares bis-Trimethylolpropan-Formal (II) | 0 | | 0 | 0 | 0 | 0 | 0 | 0,1 |
| Hochsieder | 0 | | 0 | 0 | 0 | 0,1 | 0,9 | 1,8 |

| | | | | | | | | |
|---|---|---|---|---|---|---|---|---|
| *Destillationsverlust: Einsatz (Gramm) - (Summe aus 1-6. Fraktion + Rückstand); entspricht 29,3 Gramm (0,6%) | | | | | | | | |

**Tabelle 4: Kontinuierliche destillative Aufarbeitung der wässrigen Lösung von Rückständen aus der Trimethylolpropanherstellung nach Hydrierung und Behandlung mit Ionenaustauschern**

| | | Destillation 1 8 Böden | | Destillation 2 4 Böden |
|---|---|---|---|---|
| | **Einsatz** | **Kopf** | **Sumpf *Destillation 1*** | **Sumpf *Destillation 2*** |
| Druck | [hPa] | 10 | | 2 |
| Kopf | [°C] | 156-198 | | 204 |
| Sumpf | [°C] | 185-240 | | 261 |
| Rücklaufverhältnis | | Ohne | | Ohne |
| | | | | |
| Menge [g/h] * | 1711,5 | 1221,6 | 486,6 | 432,6 |
| | | | | |
| Gaschromatographische Analyse (%): | | | | |
| Vorlauf | 1,1 | 2,1 | 0 | 0 |
| Monocycl. Formal (I) | 8,2 | 11,6 | 0 | 0 |
| Trimethylolpropan | 61,9 | 83,4 | 0,8 | 0,1 |
| Zwischenlauf I | 3,5 | 2,8 | 8,7 | 0,9 |
| Di-Trimethylolpropan | 25,0 | 0 | 87,7 | 96,7 |
| Zwischenlauf II | 0,1 | 0,1 | 0,5 | 0 |
| Lineares bis-Trimethylolpropan-Formal (II) | 0 | 0 | 0 | 0 |
| Hochsieder | 0,2 | 0 | 2,3 | 2,3 |

| | | | | |
|---|---|---|---|---|
| *Mengenbilanz [g/h]: Erste Destillation: Einsatz: 1711,5 Sumpf: 486,6 Kopf: 1221,6 Destillationsverlust: 3,3 Zweite Destillation: Einsatz: 486,6 Sumpf: 432,6 Kopf/Destillationsverlust: 54,0 | | | | |

Der erhaltene Destillationsrückstand an Di-Trimethylolpropan wurde anschließend gemäß DIN 51575, modifiziert unter Zugabe von Schwefelsäure, auf den Sulfataschegehalt hin untersucht. Die nach dem erfindungsgemäßen Verfahren erhaltenen Di-Trimethylolpropanrückstände wiesen einen Sulfataschegehalt im Bereich von 200 bis 300 ppm auf.

### Vergleichsbeispiel:

Die Hydrierung der wässrigen Lösung von Rückständen aus der Trimethylolpropandestillation wurde analog den Beispielen 1 bis 3 vorgenommen. Hingegen wurde anschließend nach Feststoffabtrennung im Gegensatz zur erfindungsgemäßen Arbeitsweise die wässrige Lösung ohne Behandlung mit den sauren und basischen Ionenaustauschern fraktioniert destilliert. Bei den erhaltenen Di-Trimethylolpropanrückständen wurde der Sulfataschegehalt ebenfalls nach dem modifizierten Verfahren gemäß DIN 51575 ermittelt. Ohne die Behandlung des wässrigen Hydrieraustrags mit Ionenaustauschern wurden Sulfataschegehalte von 1000 bis 1500 ppm ausgewiesen.

Wie die erfindungsgemäßen Beispiele belegen, kann aus den schwersiedenden Fraktionen und Rückständen, die bei der destillativen Reinigung von Trimethylolpropan anfallen, ein trimethylolpropanreicher Produktstrom destillativ abgetrennt werden während gleichzeitig im Destillationsrückstand Di-Trimethylolpropan mit einem verminderten Sulfataschegehalt verbleibt, das für technische Anwendungen eine ausreichende Qualität besitzt.

## Patentansprüche

1. Verfahren zur Gewinnung von Di-Trimethylolpropan und mit Trimethylolpropan angereicherten Produktströmen aus den schwersiedenden Fraktionen und Rückständen, die bei der destillativen Reinigung von Trimethylolpropan anfallen, **dadurch gekennzeichnet, dass** man:
(a) diese schwersiedenden Fraktionen und Rückstände vereinigt und unter Wasserzusatz eine wässrige Lösung mit einem Gehalt an organischen Komponenten von 40 bis 90 Gew.-%, bezogen auf die Gesamtmasse, herstellt;
(b) die nach Schritt a) hergestellte wässrige Lösung bei einer Temperatur von 160 bis 280°C und bei einem Druck von 1 bis 30 MPa mit Wasserstoff in Gegenwart eines Katalysators und einer aciden Verbindung, ausgewählt aus der Gruppe von protischen anorganischen Säuren, organischen Säuren und sauren Feststoffen, behandelt;
(c) die nach Schritt b) erhaltene wässrige Lösung vom Katalysator und weiteren Feststoffen, falls vorhanden, abtrennt;
(d) die nach Schritt c) erhaltene wässrige Lösung sowohl mit basischen als auch mit sauren Ionenaustauschern behandelt;
(e) aus dem nach Schritt d) erhaltenen wässrigen Eluat Wasser und vorhandene Leichtsieder entfernt; und
(f) aus dem nach Schritt e) erhaltenen Rückstand bei erhöhter Temperatur und vermindertem Druck einen mit Trimethylolpropan angereicherten Produktstrom abdestilliert, wobei Di-Trimethylolpropan als Destillationsrückstand verbleibt.

2. Verfahren gemäß Anspruch 1, **dadurch gekennzeichnet, dass** die wässrige Lösung gemäß Schritt a) bei einer Temperatur von über 50°C hergestellt wird.

3. Verfahren gemäß Anspruch 1 oder 2, **dadurch gekennzeichnet, dass** die Behandlung der wässrigen Lösung in Schritt b) mit Wasserstoff bei einer Temperatur von 180 bis 230°C und bei einem Druck im Bereich von 6 bis 20 MPa erfolgt.

4. Verfahren gemäß einem oder mehreren der Ansprüche 1 bis 3, **dadurch gekennzeichnet, dass** man als protische anorganische Säuren Phosphorsäure oder Schwefelsäure einsetzt.

5. Verfahren gemäß einem oder mehreren der Ansprüche 1 bis 3, **dadurch gekennzeichnet, dass** man als organische Säuren Ameisensäure, Essigsäure, Propionsäure oder die isomeren Buttersäuren verwendet.

6. Verfahren gemäß einem oder mehreren der Ansprüche 1 bis 3, **dadurch gekennzeichnet, dass** man als saure Feststoffe saures Aluminiumoxid, saure Zeolithe oder saure Ionenaustauscher verwendet.

7. Verfahren gemäß einem oder mehrerer der Ansprüche 1 bis 6, **dadurch gekennzeichnet, dass** man in Schritt b) als Katalysator einen Hydrierkatalysator enthaltend ein Edelmetall aus der Reihe, Ru, Rh, Pd oder Pt, oder enthaltend Co oder Ni verwendet.

8. Verfahren gemäß Anspruch 7, **dadurch gekennzeichnet, dass** der Hydrierkatalysator Aktivkohle, Aluminiumoxid, SiO₂, TiO₂, ZrO₂ oder Silikate als Trägermaterial enthält.

9. Verfahren gemäß einem oder mehreren der Ansprüche 1 bis 8, **dadurch gekennzeichnet, dass** in Schritt f) aus dem Rückstand bei einer Temperatur von bis 260°C ein mit Trimethylolpropan angereicherter Produktstrom abdestilliert wird.

## Claims

1. Process for obtaining ditrimethylolpropane and trimethylolpropane-enriched product streams from the high-boiling fractions and residues which are obtained in the distillative purification of trimethylolpropane, **characterized in that**:
(a) these high-boiling fractions and residues are combined and water is added to produce an aqueous solution having a content of organic components of 40 to 90% by weight, based on the total mass;
(b) the aqueous solution produced according to step a) is treated at a temperature of 160 to 280°C and at a pressure of 1 to 30 MPa with hydrogen in the presence of a catalyst and of an acidic compound selected from the group of protic inorganic acids, organic acids and acidic solids;
(c) the aqueous solution obtained according to step b) is removed from the catalyst and further solids, if present;
(d) the aqueous solution obtained according to step c) is treated both with basic and with acidic ion exchangers;
(e) water and low boilers present are removed from the aqueous eluate obtained according to step d); and
(f) a trimethylolpropane-enriched product stream is distilled out of the residue obtained after step e) at elevated temperature and reduced pressure, leaving ditrimethylolpropane as the distillation residue.

2. Process according to Claim 1, **characterized in that** the aqueous solution according to step a) is produced at a temperature of more than 50°C.

3. Process according to Claim 1 or 2, **characterized in that** the treatment of the aqueous solution in step b) with hydrogen is effected at a temperature of 180 to 230°C and at a pressure in the range from 6 to 20 MPa.

4. Process according to one or more of Claims 1 to 3, **characterized in that** the protic inorganic acids used are phosphoric acid or sulphuric acid.

5. Process according to one or more of Claims 1 to 3, **characterized in that** the organic acids used are formic acid, acetic acid, propionic acid or the isomeric butyric acids.

6. Process according to one or more of Claims 1 to 3, **characterized in that** the acidic solids used are acidic alumina, acidic zeolites or acidic ion exchangers.

7. Process according to one or more of Claims 1 to 6, **characterized in that** the catalyst used in step b) is a hydrogenation catalyst comprising a noble metal from the group of Ru, Rh, Pd and Pt, or comprising Co and Ni.

8. Process according to Claim 7, **characterized in that** the hydrogenation catalyst comprises activated carbon, alumina, SiO₂, TiO₂, ZrO₂ or silicates as support material.

9. Process according to one or more of Claims 1 to 8, **characterized in that**, in step f), a trimethylolpropane-enriched product stream is distilled out of the residue at a temperature of up to 260°C.

## Revendications

1. Procédé d'obtention de di-triméthylolpropane et de courants de produits enrichis en triméthylolpropane à partir des fractions et des résidus de point d'ébullition élevé formés lors de la purification par distillation de triméthylolpropane, **caractérisé en ce que** :
(a) ces fractions et résidus de point d'ébullition élevé sont réunis et une solution aqueuse ayant une teneur en composants organiques de 40 à 90 % en poids, par rapport à la masse totale, est fabriquée par ajout d'eau ;
(b) la solution aqueuse fabriquée selon l'étape a) est traitée à une température de 160 à 280 °C et à une pression de 1 à 30 MPa avec de l'hydrogène en présence d'un catalyseur et d'un composé acide choisi dans le groupe constitué par les acides inorganiques protiques, les acides organiques et les solides acides ;
(c) la solution aqueuse obtenue selon l'étape b) est séparée du catalyseur et des autres solides, s'ils sont présents ;
(d) la solution aqueuse obtenue selon l'étape c) est traitée avec des échangeurs d'ions aussi bien basiques qu'acides ;
(e) l'eau et les composés de point d'ébullition faible présents sont éliminés de l'éluat aqueux obtenu selon l'étape d) ; et
(f) un courant de produits enrichi en triméthylolpropane est séparé par distillation à température élevée et pression réduite à partir du résidu obtenu selon l'étape e), le di-triméthylolpropane restant en tant que résidu de distillation.

2. Procédé selon la revendication 1, **caractérisé en ce que** la solution aqueuse selon l'étape a) est fabriquée à une température supérieure à 50 °C.

3. Procédé selon la revendication 1 ou 2, **caractérisé en ce que** le traitement de la solution aqueuse à l'étape b) avec de l'hydrogène a lieu à une température de 180 à 230 °C et à une pression dans la plage allant de 6 à 20 MPa.

4. Procédé selon une ou plusieurs des revendications 1 à 3, **caractérisé en ce que** l'acide phosphorique ou l'acide sulfurique est utilisé en tant qu'acide inorganique protique.

5. Procédé selon une ou plusieurs des revendications 1 à 3, **caractérisé en ce que** l'acide formique, l'acide acétique, l'acide propionique ou les acides butyriques isomères sont utilisés en tant qu'acides organiques.

6. Procédé selon une ou plusieurs des revendications 1 à 3, **caractérisé en ce qu'**un oxyde d'aluminium acide, une zéolithe acide ou un échangeur d'ions acide est utilisé en tant que solide acide.

7. Procédé selon une ou plusieurs des revendications 1 à 6, **caractérisé en ce qu'**un catalyseur d'hydrogénation contenant un métal noble de la série constituée par Ru, Rh, Pd ou Pt, ou contenant Co ou Ni, est utilisé en tant que catalyseur à l'étape b).

8. Procédé selon la revendication 7, **caractérisé en ce que** le catalyseur d'hydrogénation contient du charbon actif, de l'oxyde d'aluminium, SiO₂, TiO₂, ZrO₂ ou des silicates en tant que matériau support.

9. Procédé selon une ou plusieurs des revendications 1 à 8, **caractérisé en ce qu'**un courant de produits enrichi en triméthylolpropane est séparé par distillation à une température de jusqu'à 260 °C à partir du résidu à l'étape f).
